# EUROPEAN PATENT APPLICATION

(11) **EP 4 310 513 A1**
(43) Date of publication of application: **24.01.2024**
(21) Application number: 21931711.2
(22) Date of filing: 26.11.2021
(51) Int. Cl.: G01N 37/00, C12M 1/26, C12N 13/00, B01J 19/00, G01N 1/04, G01N 27/02

(54) **MICROFLUIDIC CHIP AND MANUFACTURING METHOD THEREOF**

(30) Priority: 19.03.2021 JP 2021045561
(71) Applicant: SCREEN Holdings Co., Ltd., Kyoto-shi, Kyoto 602-8585 (JP)
(72) Inventor: URAKAWA, Satoshi, Kyoto-shi, Kyoto 602-8585 (JP)
(74) Representative: Goddar, Heinz J.
(86) International application number: PCT/JP2021/043457
(87) International publication number: WO 2022/195968

(57) **Abstract**

A technique for appropriately protecting electrodes of a microfluidic chip is provided. A microfluidic chip (1) includes a substrate (10), electrodes (31), insulation films (33), and a fluid holding part (20). The electrodes (31) are disposed on an upper surface of the substrate (10). The insulation films (33) cover upper surfaces of the respective electrodes (31). The insulation films (33) are comprised of an oxide film obtained by oxidizing a metal contained in the electrodes (31) or a nitride film obtained by nitriding the metal contained in the electrodes (31). The fluid holding part (20) is capable of accommodating a fluid over the insulation films (33).

## Description

### Technical Field

The present invention relates to a microfluidic chip and a method of manufacturing the same.

### Background Art

In the field of regenerative medicine, importance has been placed on sorting techniques for separating and trapping biological particles (particles in living bodies) such as cells, and a sorting device that uses a dielectrophoretic force has been known as one of such techniques (for example, Patent Literature 1). This type of sorting device includes, for example, a channel through which biological particles flow together with a sample liquid (such as a culture solution), and electrodes for sorting the biological particles. The sorting device applies an alternating voltage to the electrodes to generate an electric field in the liquid flowing through the channel, thereby electrically sorting the biological particles.

### Citation List

### Patent Literature

Patent Literature 1: International Publication No. 2014/207617

### Summary of Invention

### Technical Problem

When a voltage at such a level as to move the biological particles is applied to the electrodes, an electrochemical reaction occurs between the metal that constitutes the electrodes and a liquid interface, so that the electrodes can be easily electrolyzed. For this reason, there are cases in which an insulating protective film is formed by a deposition process to protect the electrodes.

However, the insulating protective film acts as a reactance (pseudo-resistance) under the alternating voltage to decrease the level of voltage output from the electrodes. This can significantly decrease sorting performance if the deposited insulating protective film is relatively thick (e.g., if the film thickness of the electrodes is 100 nm and the film thickness of the insulating protective film is 200 nm). On the other hand, if the deposited insulating protective film is thin (e.g., 100 nm or less), surfaces of the electrodes are prone to be exposed due to film deposition variations. In particular, sidewall portions of the electrodes are prone to be exposed if unidirectional deposition such as sputtering is performed.

It is an object of the present invention to provide a technique for appropriately protecting electrodes of a microfluidic chip.

### Solution to Problem

To solve the aforementioned problem, a first aspect is intended for a microfluidic chip comprising: a substrate; an electrode disposed on a surface on one side of the substrate; an insulation film covering a surface on one side of the electrode and comprised of an oxide film obtained by oxidizing a metal contained in the electrode or a nitride film obtained by nitriding the metal; and a fluid holding part capable of accommodating a fluid on the one side of the insulation film.

A second aspect is intended for the microfluidic chip of the first aspect, wherein the fluid holding part accommodates the fluid, with the fluid in contact with the insulation film.

A third aspect is intended for the microfluidic chip of the first or second aspect, wherein the electrode includes, in order toward the one side, an electrically conductive layer containing a first metal having electrical conductivity, and a surface layer containing a second metal different from the first metal, and wherein the insulation film is a film of an oxide or nitride of the second metal contained in the surface layer.

A fourth aspect is intended for the microfluidic chip of the third aspect, wherein the second metal is titanium, indium, tin, copper, molybdenum, silver, chromium, tantalum, tungsten, silicon, or a metal compound composed thereof.

A fifth aspect is intended for the microfluidic chip of any one of the first to fourth aspects, wherein the fluid holding part has a channel through which the fluid flows, and wherein the channel is positioned on the one side of the insulation film.

A sixth aspect is intended for the microfluidic chip of the fifth aspect, wherein the channel includes a main channel having a first end portion and a second end portion, the fluid flowing from the first end portion toward the second end portion, and a plurality of sub-channels connected to the second end portion of the main channel, the fluid flowing through the sub-channels, and wherein the electrode applies a voltage for separating particles in the fluid flowing through the main channel into one of the sub-channels.

A seventh aspect is intended for a method of manufacturing a microfluidic chip, which comprises the steps of: a) preparing a substrate having an electrode on a surface on one side thereof; b) oxidizing or nitriding a metal contained in the electrode in the substrate to thereby form an insulation film that is an oxide or nitride film on a surface on the one side of the electrode; and c) forming a fluid holding part capable of accommodating a fluid on one side of the insulation film, the step c) being performed after the step b).

An eighth aspect is intended for the method of manufacturing a microfluidic chip of the seventh aspect, wherein the step b) is the step of forming the insulation film by means of a thermal oxidation method or a wet oxidation method.

A ninth aspect is intended for the method of manufacturing a microfluidic chip of the seventh or eighth aspect, wherein the electrode includes, in order toward the one side, an electrically conductive layer containing a first metal having electrical conductivity, and a surface layer containing a second metal different from the first metal, and wherein the step b) is the step of forming the insulation film by oxidizing or nitriding the second metal of the surface layer.

### Advantageous Effects of Invention

According to the microfluidic chip of the first aspect, the insulation film is formed by the oxidation or nitriding process of the electrode surface. This achieves the protection of the electrode more easily as compared with the formation of the insulation film by means of a deposition process.

According to the microfluidic chip of the second aspect, the electrode is appropriately protected at lower costs and with fewer process steps as compared with the formation of a protective film between the electrode and the fluid holding part by means of a deposition process.

According to the microfluidic chip of the third aspect, the electrical conductivity of the electrode is ensured by the electrically conductive layer of the first metal. This allows the selection of the second metal suitable for the formation of the oxide or nitride film.

According to the microfluidic chip of the fourth aspect, the electrode is effectively protected by the oxide or nitride film of the second metal.

According to the microfluidic chip of the fifth aspect, the voltage is applied to the electrode while the fluid flows through the channel, whereby the voltage is applied to the fluid.

According to the microfluidic chip of the sixth aspect, the electrode applies the voltage for separating particles in the fluid flowing through the main channel into one of the sub-channels. This achieves the sorting of the particles in the fluid.

According to the method of manufacturing a microfluidic chip of the seventh aspect, the insulation film is formed by the oxidation or nitriding process of the electrode surface. This achieves the protection of the electrode more easily as compared with the formation of the insulation film by means of a deposition process.

According to the method of manufacturing a microfluidic chip of the eighth aspect, the insulation film is formed with ease because the surface of the electrode is oxidized with ease.

According to the method of manufacturing a microfluidic chip of the ninth aspect, the electrical conductivity of the electrode is ensured by the electrically conductive layer of the first metal. This allows the selection of the second metal suitable for the formation of the oxide or nitride film.

### Brief Description of Drawings

[fig. 1] Fig. 1 is a plan view of a microfluidic chip according to an embodiment.
[fig. 2] Fig. 2 is a plan view and a sectional view which show a voltage application part of the microfluidic chip shown in Fig. 1.
[fig. 3] Fig. 3 is a view conceptually showing the formation of an insulation film on a surface of an electrode.
[fig. 4] Fig. 4 shows changes in impedance with and without insulation films and a protective film.
[fig. 5] Fig. 5 shows changes in capacitance with and without the insulation films.
[fig. 6] Fig. 6 is a plan view showing electrodes on which an endurance test was conducted.

### Description of Embodiments

An embodiment according to the present invention will now be described with reference to the accompanying drawings. Components described in the embodiment are merely illustrative, and there is no intention to limit the scope of the present invention only thereto. In the drawings, the dimensions of components and the number of components are shown in exaggeration or in simplified form, as appropriate, for the sake of easier understanding in some cases.

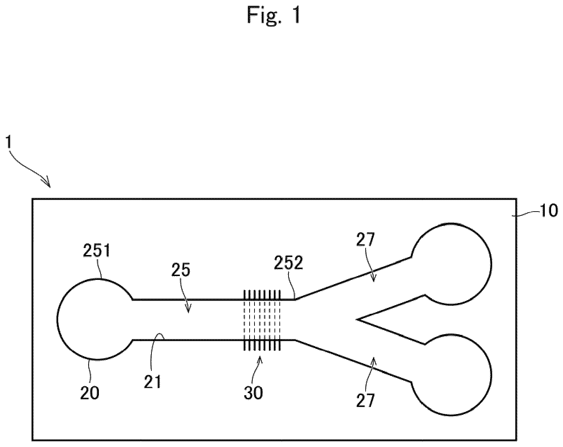

Fig. 1 is a plan view of a microfluidic chip 1 according to the embodiment. The microfluidic chip 1 is a device that applies a voltage to a fluid. The microfluidic chip 1 is, for example, a cell sorter for separating and trapping biological particles such as cells. The microfluidic chip 1 includes a substrate 10, a fluid holding part 20, and a voltage application part 30. The components of the microfluidic chip 1 will be described below.

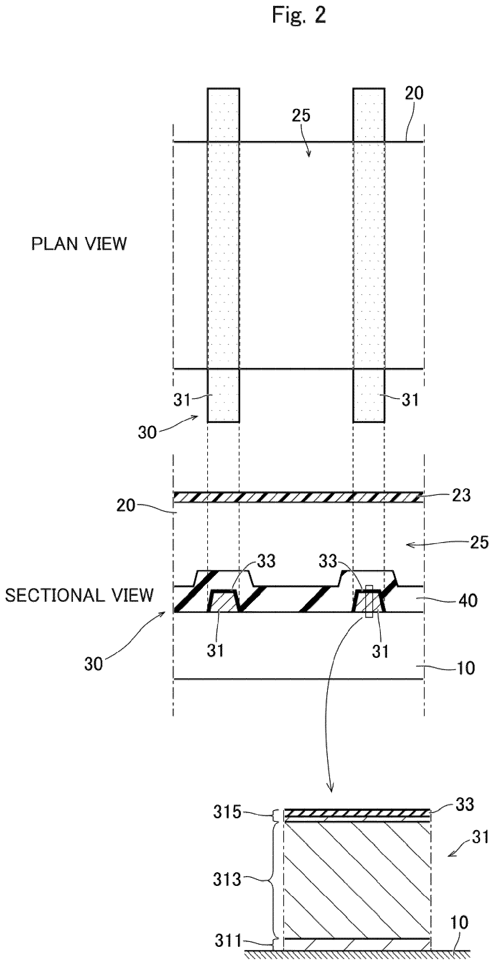

The substrate 10 is a glass substrate made of quartz, for example. The fluid holding part 20 has a structure capable of accommodating a fluid (such as a liquid containing biological particles), and is made of resin or quartz glass. The fluid holding part 20 is disposed over the substrate 10 (with reference to Fig. 2). The fluid holding part 20 has a side wall portion 21 and a ceiling portion 23 (with reference to Fig. 2). The side wall portion 21 and the ceiling portion 23 are portions for partitioning the space that accommodates the fluid, and form walls standing with respect to a surface of the substrate 10. The ceiling portion 23 is a portion for closing the upper side of the space that accommodates the fluid.

As shown in Fig. 1, the fluid holding part 20 constitutes channels through which a fluid flows. Specifically, the fluid holding part 20 has a main channel 25 and two sub-channels 27. The main channel 25 has a first end portion 251 and a second end portion 252. The first end portion 251 is able to be supplied with a liquid. The liquid supplied to the first end portion 251 flows toward the second end portion 252.

An end portion of each of the two sub-channels 27 is connected to the second end portion 252 of the main channel 25. The main channel 25 and the two sub-channels 27 are coupled for flow of a liquid therebetween.

The voltage application part 30 includes a plurality of electrodes 31 arranged in a comb tooth shape. As shown in Fig. 2, the electrodes 31 extend so as to traverse the main channel 25 of the fluid holding part 20.

An alternating voltage for separating biological particles in the liquid is applied to each of the electrodes 31 of the voltage application part 30. When the alternating voltage is applied, an electric field for sorting is generated around each of the electrodes 31. This electric field for sorting acts on the biological particles in the liquid flowing through the main channel 25, whereby the biological particles in the liquid are fed to either one of the two sub-channels 27.

Fig. 2 is a plan view and a sectional view which show the voltage application part 30 of the microfluidic chip 1 shown in Fig. 1. As shown in Fig. 2, the electrodes 31 of the voltage application part 30 are disposed on the upper surface of the substrate 10. The electrodes 31 are made of an electrically conductive metal. The concept of "metal" used herein includes alloys.

Each of the electrodes 31 shown in Fig. 2 has a multi-layer structure, and includes an adhesion layer 311, an electrically conductive layer 313, and a surface layer 315 in the order from the bottom (the substrate 10 side) to the top. The adhesion layer 311 is a layer provided for intimate contact between the surface of the substrate 10 and the electrodes 31, and contains, for example, titanium. The electrically conductive layer 313 is made of an electrically conductive metal (such as aluminum). The surface layer 315 is in the topmost position of each of the electrodes 31, and contains, for example, titanium. The multi-layer structure of the electrodes 31 is formed, for example, by deposition using vacuum evaporation. The thicknesses of the respective layers of the electrodes 31 are not particularly limited, but may be 10 nm in thickness of the adhesion layer 311, 100 nm in thickness of the electrically conductive layer 313, and 10 nm in thickness of the surface layer 315.

In the electrodes 31, the adhesion layer 311 that contacts the substrate 10 is made of titanium, whereby the electrodes 31 are brought into intimate contact with the substrate 10. In particular, when the substrate 10 is a glass substrate, the electrodes 31 are satisfactorily brought into intimate contact with the substrate 10. The surface layer 315 in each of the electrodes 31 is provided for the formation of an insulation film 33 to be described later. The metal (a first metal) of the electrically conductive layer 313 is preferably higher in electrical conductivity than the metal (a second metal) of the surface layer 315 and the metal of the adhesion layer 311. Examples of the metal of the surface layer 315 usable herein may include aluminum, indium, tin, copper, molybdenum, silver, chromium, tantalum, tungsten, silicon, and metal compounds composed thereof, in addition to titanium.

The electrodes 31 are not required to have a multi-layer structure, but may have a single-layer structure. The electrodes 31 are not required to contain more than one type of metal, but may contain only one type of metal. When the electrodes 31 are made of one type of metal, for example, aluminum, titanium, indium, tin, copper, molybdenum, silver, chromium, tantalum, tungsten, silicon, or a metal compound composed thereof may be employed.

As shown in Fig. 2, an upper surface of each of the electrodes 31 is covered with the insulation film 33. The insulation film 33 is comprised of an oxide film obtained by oxidizing the metal constituting the electrodes 31 or a nitride film obtained by nitriding the metal constituting the electrodes 31. That is, the insulation film 33 is expressed by M_{X}O_{Y} for the oxide film or by M_{X}N_{Y} for the nitride film (X and Y are the number of atoms) where "M" is the symbol of the element of the electrode metal.

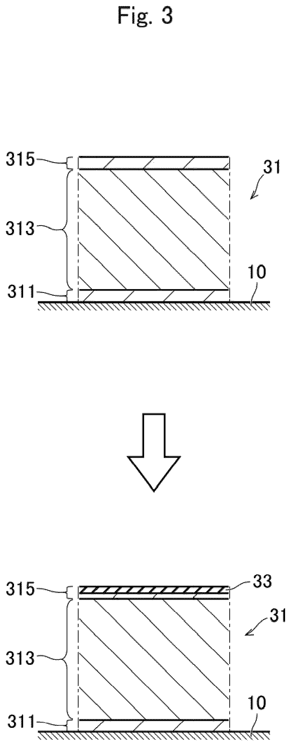

Fig. 3 is a view conceptually showing the formation of the insulation film 33 on the surface of an electrode 31. As shown in Fig. 3, the insulation film 33 is formed on the surface of the electrode 31 by performing an oxidation or nitriding process on the surface layer 315 of the electrode 31. For oxidation of the surface layer 315, for example, a thermal oxidation method (a dry thermal oxidation method or a hydrothermal oxidation method) or a wet oxidation method is applicable. For example, when the surface layer 315 of the electrode 31 is made of titanium with a thickness of 10 nm, the titanium of the surface layer 315 is oxidized by treatment for 2 hours using an autoclave with a water filling ratio of 5 to 10% to the internal volume of the autoclave and a temperature of 130 to 140°C. For nitriding of the surface layer 315, a nitriding process that exposes the surface layer 315 to a nitriding atmosphere at high temperature may be employed.

As shown in Fig. 2, the surface of the substrate 10 including the electrodes 31 with the insulation films 33 formed thereon may be covered with a protective film 40. The protective film 40 is, for example, a silicon oxide film (SiOx film) deposited by sputtering. The thickness of the protective film 40 is preferably not greater than 200 nm. As shown in Fig. 2, the protective film 40 forms a bottom surface of the channel formed by the fluid holding part 20. In other words, an upper surface of the protective film 40 is a surface that contacts the liquid flowing in the fluid holding part 20. When the insulation films 33 are an oxide film of titanium (TiOx), the intimate contact between the insulation films 33 and the protective film 40 (silicon oxide film) is improved.

It is not essential that the microfluidic chip 1 includes the protective film 40. In other words, the microfluidic chip 1 need not include the protective film 40. In this case, the surface of the substrate 10 and the insulation films 33 positioned on the surfaces of the electrodes 31 form the bottom surface of the fluid holding part 20. Thus, when the fluid holding part 20 contains the liquid, the surface of the substrate 10 and the insulation films 33 come in contact with the liquid. Even in the absence of the protective film 40, the insulation films 33 prevent the electrodes 31 from coming in direct contact with the liquid. This suppresses the electrolysis of the electrodes 31 at the time of voltage application. Thus, when the protective film 40 is not formed by the deposition process, the microfluidic chip 1 is produced at lower costs and with fewer process steps because the cost and the number of process steps for the formation of the protective film 40 are dispensed with.

The fluid holding part 20 is attached to the upper surface of the substrate 10 after the insulation films 33 are formed on the surfaces of the electrodes 31. This places the fluid holding part 20 over the insulation films 33. If the protective film 40 is formed on the upper surface of the substrate 10, the fluid holding part 20 may be attached to the upper surface of the substrate 10 after the protective film 40 is formed.

### <Change in Electrical Properties depending on Insulation films>

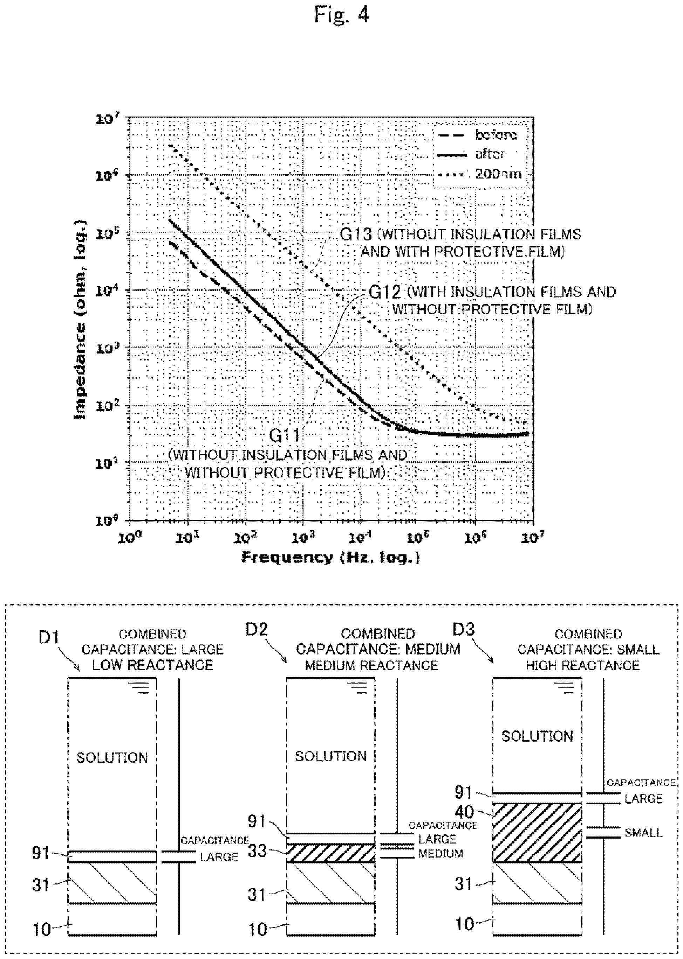

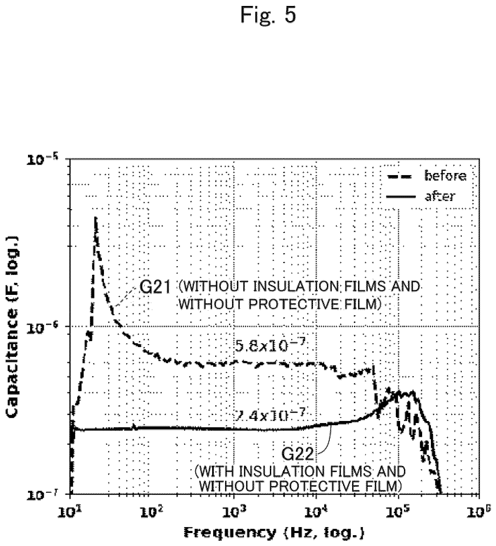

Next, changes in electrical properties of the microfluidic chip 1 with and without the insulation films 33 and the protective film 40 will be described with reference to Figs. 4 and 5.

Fig. 4 shows changes in impedance with and without the insulation films 33 and the protective film 40. In Fig. 4, the ordinate represents impedance, and the abscissa represents frequency. Fig. 5 shows changes in capacitance with and without the insulation films 33. In Fig. 5, the ordinate represents capacitance, and the abscissa represents frequency. Figs. 4 and 5 show the results of measuring the electrical properties (impedance and capacitance) of the microfluidic chip 1 by applying an alternating voltage to the electrodes 31 by means of an LCR meter. A high conductivity liquid is used as a sample liquid contained in the fluid holding part 20 for the purpose of making the influence of the liquid on measurement values closer to zero.

In Fig. 4, a curve G11 represents measurement results obtained without the insulation films 33 and without the protective film 40. In Fig. 4, a curve G12 represents measurement results obtained with the insulation films 33 and without the protective film 40. In Fig. 4, a curve G13 represents measurement results obtained without the insulation films 33 and with the protective film 40 (a SiOx film with a thickness of 200 nm). Cross-sectional structures of devices D1, D2, and D3 corresponding to the respective curves G11, G12, and G13 are conceptually shown in Fig. 4. In Fig. 5, a curve G21 represents measurement results obtained without the insulation films 33 and without the protective film 40. In Fig. 5, a curve G22 represents measurement results obtained with the insulation films 33 and without the protective film 40.

As shown in Fig. 4, the impedance shifts to a high-frequency side in the case with the protective film 40 (the curve G13) as compared with the cases without the protective film 40 (the curves G11 and G12). This shift indicates that the reactance is higher due to a lower current generated when the alternating voltage is applied. In other words, the higher reactance of the protective film 40 causes a voltage drop in the protective film 40, which in turn makes it difficult to sweep the voltage efficiently through the liquid. For efficient sweep of the voltage, it is preferable that the thickness of the protective film 40 is as thin as possible or that the protective film 40 is not provided.

As shown in Fig. 4, in the case without the insulation films 33 (the curve G11), the reactance is lowered because the large capacitance of an electric double layer 91 formed at a solution interface with the electrodes 31 predominates, which in turn results in a low impedance. On the other hand, in the case with the insulation films 33 (the curve G12), the impedance shifts to the high-frequency side. This indicates that a capacitance component derived from the insulation films 33 is formed, but this is because the combined capacitance with the aforementioned electric double layer 91 is smaller than that without the insulation films 33. In other words, the reactance is higher because the capacitance component derived from the insulation films 33 is small in capacitance, which in turn results in a high impedance.

As shown in Fig. 5, in the case without the insulation films 33 (the curve G21), an increase in capacitance is found in a low-frequency region where a DC component starts appearing. This increase is considered to result from electrolysis that occurs because the electrodes 31 are exposed to the liquid and the resulting increase in current. On the other hand, in the case with the insulation films 33 (the curve G22), the capacitance component exhibits a substantially constant value independently of frequency. In other words, the electrolysis of the electrodes 31 is considered to be suppressed by the presence of the insulation films 33.

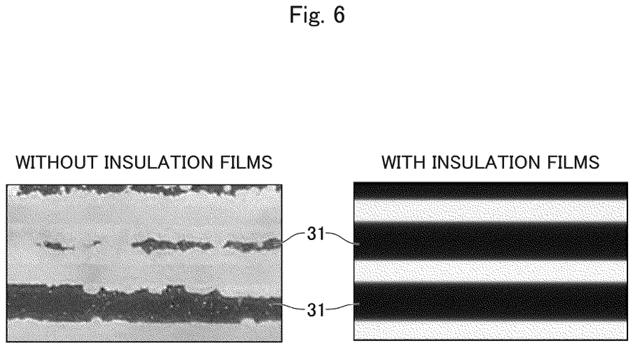

Fig. 6 is a plan view showing the electrodes 31 on which an endurance test was conducted. In the endurance test, a predetermined alternating voltage (2 V, 10 Hz) was applied to the electrodes 31, with a saturated saline solution stored in the fluid holding part 20. The electrodes 31 without the insulation films 33 are shown in the left-hand part of Fig. 6, and the electrodes 31 with the insulation films 33 are shown in the right-hand part of Fig. 6. In both cases, the protective film 40 is not provided.

As shown in Fig. 6, it is found that the electrodes 31 are significantly damaged in the case without the insulation films 33 (the left-hand part), as compared with the case with the insulation films 33 (the right-hand part). As is apparent from these results, the electrolysis of the electrodes 31 due to the contact with the liquid is appropriately suppressed by forming the insulation films 33 on the surfaces of the electrodes 31.

In the aforementioned embodiment, the electrodes 31 are disposed so as to generate a force that separates the biological particles moving through the main channel 25 into one of the two sub-channels 27. However, applications of the electrodes are not limited to this. For example, the electrodes may be disposed so as to allow the electrical properties (e.g., dielectric spectrum) of the particles in the liquid to be measured.

While the invention has been described in detail, the foregoing description is in all aspects illustrative, and the invention is not limited thereto. It is therefore understood that numerous modifications and variations not illustrated can be devised without departing from the scope of the invention. The components described in the aforementioned embodiment and in the various modifications may be combined together or dispensed with, as appropriate, unless the components are inconsistent with each other.

### Reference Signs List

1 Microfluidic chip
10 Substrate
20 Fluid holding part
25 Main channel
27 Sub-channels
31 Electrodes
33 Insulation films
251 First end portion
252 Second end portion
313 Electrically conductive layer
315 Surface layer

## Claims

1. A microfluidic chip comprising:
a substrate;
an electrode disposed on a surface on one side of said substrate;
an insulation film covering a surface on one side of said electrode and comprised of an oxide film obtained by oxidizing a metal contained in said electrode or a nitride film obtained by nitriding said metal; and
a fluid holding part capable of accommodating a fluid on said one side of said insulation film.

2. The microfluidic chip according to Claim 1,
wherein said fluid holding part accommodates said fluid, with said fluid in contact with said insulation film.

3. The microfluidic chip according to Claim 1 or 2,
wherein said electrode includes, in order toward said one side,
an electrically conductive layer containing a first metal having electrical conductivity, and
a surface layer containing a second metal different from said first metal, and
wherein said insulation film is a film of an oxide or nitride of said second metal contained in said surface layer.

4. The microfluidic chip according to Claim 3,
wherein said second metal is titanium, indium, tin, copper, molybdenum, silver, chromium, tantalum, tungsten, silicon, or a metal compound composed thereof.

5. The microfluidic chip according to any one of Claims 1 to 4,
wherein said fluid holding part has a channel through which said fluid flows, and
wherein said channel is positioned on said one side of said insulation film.

6. The microfluidic chip according to Claim 5,
wherein said channel includes
a main channel having a first end portion and a second end portion, said fluid flowing from said first end portion toward said second end portion, and
a plurality of sub-channels connected to said second end portion of said main channel, said fluid flowing through said sub-channels, and
wherein said electrode applies a voltage for separating particles in said fluid flowing through said main channel into one of said sub-channels.

7. A method of manufacturing a microfluidic chip, comprising the steps of:
a) preparing a substrate having an electrode on a surface on one side thereof;
b) oxidizing or nitriding a metal contained in said electrode in said substrate to thereby form an insulation film that is an oxide or nitride film on a surface on said one side of said electrode; and
c) forming a fluid holding part capable of accommodating a fluid on one side of said insulation film, said step c) being performed after said step b).

8. The method of manufacturing a microfluidic chip according to Claim 7,
wherein said step b) is the step of forming said insulation film by means of a thermal oxidation method or a wet oxidation method.

9. The method of manufacturing a microfluidic chip according to Claim 7 or 8,
wherein said electrode includes, in order toward said one side,
an electrically conductive layer containing a first metal having electrical conductivity, and
a surface layer containing a second metal different from said first metal, and
wherein said step b) is the step of forming said insulation film by oxidizing or nitriding said second metal of said surface layer.
